# EUROPEAN PATENT SPECIFICATION

(11) **EP 3 061 746 B1**
(45) Date of publication and mention of the grant of the patent: **07.12.2016**
(21) Application number: 15156234.5
(22) Date of filing: 24.02.2015
(51) Int. Cl.: C07C 303/20, C07C 309/17, C08K 5/134

(54) **HYDROPHILIC ANTIOXIDANT AND PROCESS FOR THE PREPARATION OF THE SAME**
HYDROPHILES ANTIOXIDANS UND VERFAHREN ZUR HERSTELLUNG DAVON
ANTIOXYDANT HYDROPHILE ET PROCÉDÉ POUR LA PRÉPARATION DE CELUI-CI

(43) Date of publication of application: 31.08.2016
(73) Proprietor: Founder Fine Chemical Industry Co., Ltd., Yilan County 269 (TW); Wang, Tung-Chi, Yilan County 269 (TW); Yeh, Ching-Yu, Yilan County 269 (TW)
(72) Inventor: Chen, Ya-Ling, 269 Yilan County (TW); Liou, Shih-Pin, 269 Yilan County (TW); Lin, Min-Shyan, 269 Yilan County (TW)
(74) Representative: dompatent von Kreisler Selting Werner - Partnerschaft von Patent- und Rechtsanwälten mbB

(56) References cited:
- EP-A1- 0 608 089
- JP-A- 2002 293 876
- US-A- 3 201 252

## Description

### Field of the Invention

The present invention relates to a method for preparing a hydrophilic antioxidant, in particular relates to a method for preparing hydrophilic antioxidants containing methyl 3-(3,5-di-tert-butyl-4-hydroxyphenyl)propionate groups / poly alkylene glycol bridge groups / carboxylic acid groups/ sulfonic acid groups.

### Description of Prior Art

The plastic polymer chains are easily broken under UV light and heat to generate active radicals. The active radicals react with oxygen to form peroxide radical, then further reacts with the remaining polymer chain to generate oxidized products and new active radicals. The active radicals continue to cause radical chain reactions, and as a result the plastic is prone to aging.

Methyl 3-(3,5-di-tert-butyl-4-hydroxyphenyl)propionates (a.k.a. 3,5-methyl propionate) are primary antioxidants, which are hindered phenol molecules, frequently used in plastics. The antioxidants has the advantages of high molecular weight, low volatility, high antioxidant characteristic and low toxicity, yet such antioxidants are limited by poor water solubility and is not able to used alone in aqueous phase systems.

Water soluble antioxidants are normally natural products, for example, vitamin C, vitamin B, tea polyphenols, which are widely used in medical researches among biological systems. Such natural water soluble antioxidants do not contain 3,5-methyl propionate groups.

The hindered phenol small molecules of 3,5-methyl propionates are, for example, 3,5-di-tert-butyl-4-hydroxybenzoic acid, 3,5-di-tert-butyl-4-hydroxy phenylacetic acid, 3,5-di-tert-butyl-4-hydroxyphenylpropionic acid. Such hindered phenol small molecules are water soluble and has oxidation resistance, however, are easily removed from the substrate surface (via volatilization or dissipation) upon heating under high temperature, in addition, the antioxidant characteristic is reduced due to decomposition caused by material aging. Subsequently, such small molecule antioxidants are not effective to prevent substrates from aging in a long term.

PEGs, poly(ethylene glycol) are non-toxic, non-irritating and have good water solubility, are nonionic surfactant and has good inter-solubility with many organic materials. PEG are widely applied in cosmetics, pharmaceutical, chemical fiber, rubber, plastics, paper, paint, electroplating, pesticides, metals processing, bio-engineering and food processing industries.

The patent US4,032,562 disclosed that mono-substituted group transesterification is carried out with 3,5-methyl propionate and PEG and 3,5-methyl propionate / PEG mono-substituted group esterification product is obtained, for example, 23-hydroxy-3,6,9,12, 15,18,21-heptaoxatricos 3-(3,5-di-tert-butyl-4-hydroxyphenyl)propionate. Said structure introduces hydrophilic polymer PEG. Though the molecular weight increases and the low volatility issue is improved, yet said structure is only slightly soluble (5.8g/L @pH =1∼10, the values are based on theories). Similar synthesis reactions are disclosed in US3285855, US3441575, US5696281, DE2231671 and CN100430366C, where the products are oil soluble.

The di-substituted group transesterification is carried out with PEG and 3,5-methyl propionate and di-substituted group esterification products of 3,5-methyl propionate / PEG is obtained, for example, triethylene glycol [bis(3-tert-butyl-4-hydroxy-5-methylphenyl)propionate, diethylene glycol [bis 3-(3,5-di-tert-butyl-4-hydroxyphenyl)propionate)], triethylene glycol [bis[3-(3,5-di-tert-butyl-4-hydroxyphenyl)propionate], tetraethylene glycol [bis[3-(3,5-di-tert-butyl-4-hydroxyphenyl)propionate], and nonaethylene glycol [bis[3-(3,5-di-tert-butyl-4-hydroxyphenyl)propionate]. Such structure contains two 3,5methyl ester groups, and has preferred oxidation resistance and less volatility compare to mono-substituted group products. Nonetheless, the water solubility of the products is worse and the products are not applicable to aqueous phase systems.

EP 0608089 A1 discloses alkylene glycol monoether and 3,5 methyl ester groups which undergo transesterification to obtain a series of antioxidants that have good compatibility with resins. An ester compound can be obtained from the reaction: 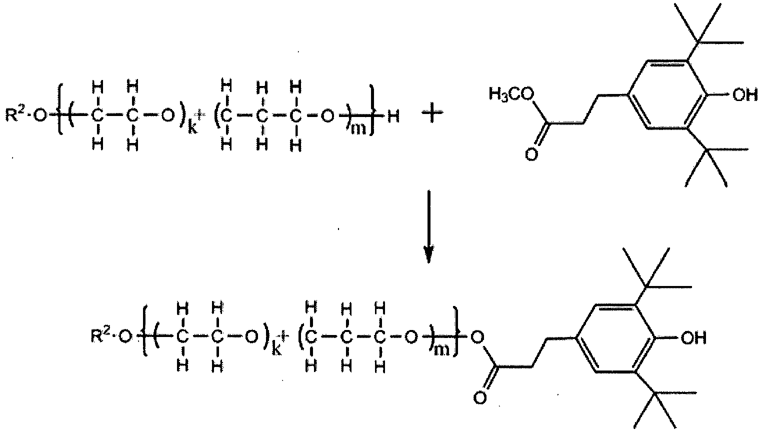 wherein:
R² represents an alkyl group having from 8 to 24 carbon atoms; k is 0 or an integer from 1 to 10; and m is 0 or an integer from 1 to 5; provided that the total of (k + m) is greater than 0 and not greater than 10.

The total number of ethyleneoxy groups and propyleneoxy groups is up to 10 and R² is a hydrophobic long carbon chain.

The German periodical Die Angewandte Makromolekulare Chemie, 260, 77-81 (1998) disclosed the epoxide group-opening is carried out with 3,5-di-tert-butyl-4-hydroxyphenylpropionic acid and 11-[(2*S*,3*R*)-3-pentyl-2-oxiranyl]-,(9*Z*) -9-undecenoic acid and (9Z)-13-(3-(3,5-di-tert-butyl-4-hydroxyphenyl)propanoyloxy)-12-hydroxyoctadec-9-enoic acid, which is the 3,5-methyl propionate mono-substituted group containing carboxylic acid group, is obtained. The antioxidant has a hydrophilic carboxylic acid group, but is still slightly soluble.

Disodium sulfosuccinate of monolauryl alcohol polyethylene is a common surfactant. Disodium sulfosuccinate of monolauryl alcohol polyethylene has two kinds of hydrophilic groups of cationic groups comprising carboxylic acid group/ sulfonic acid group and nonionic group comprising polyglycol ether, so that it can have double surface chemical properties of cationic surfactant and nonionic surfactant with excellent dispersion, wetting, clean and emulsion properties. In the fields of micro-emulsion polymerization (Langmuir (2001), 17(17), 5388-5397), detergent such as daily shampoo (DE 1768556, US 2438092), coating aids of film gelatin (US 3201252) and collector used in a froth flotation of ores(US 4192739), disodium sulfosuccinate of monolauryl alcohol polyethylene is widely used. It has been disclosed that the structures of disodium sulfosuccinate of monolauryl alcohol polyethylene have straight, branched, cyclic or aromatic alkyl group but disodium sulfosuccinate of monolauryl alcohol polyethylene comprising 3,5-methyl propionate of antioxidant characteristic has not been disclosed.

In conclusion, the anti-oxidant molecules containing 3,5-methyl propionate groups with low volatility, good water solubility have not been disclosed, accordingly the present invention is dedicated to synthesize anti-oxidants with above mentioned characteristics.

### SUMMARY OF THE INVENTION

A primary objective of the present invention is to provide an antioxidant comprising 3,5-methyl propionate groups, which has low volatility and high water solubility.

In order to achieve the above objective, the invention discloses a compound (1) synthesized with 3,5-methyl propionate groups, which has low volatility and high water solubility. 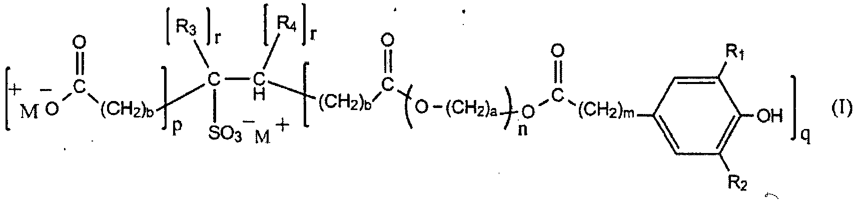
wherein R₁ and R₂ can be mutually the same or different C1∼C18 straight chain or branched-chain alkyl groups;
a is an integer of 2 to 6;
b is 0, 1, 2, 3 or 4;
m is 1 or 2;
n is an integer of 2 to 500, and preferably n is an integer of 5 to 300;
R3 and R4 can be mutually the same or different C1∼C6 straight chain or branched-chain alkyl groups or hydrogen;
M is an IA metal ions or hydrogen atom;
p is 1, 2 or 3;
q is 1, 2 or 3; and
r is 0 or 1, and p, q and r satisfying the equation p + q + 2r = 4.

The other objective of the present invention is to provide a method for preparing the compound of formula (1), wherein the compound of formula (1) is synthesized with three reaction steps:
In the first step, under the catalyzing reaction with the catalyzer, an intermediate (4) is obtained by mono-substituted group transesterification of a compound (2) and a compound (3).

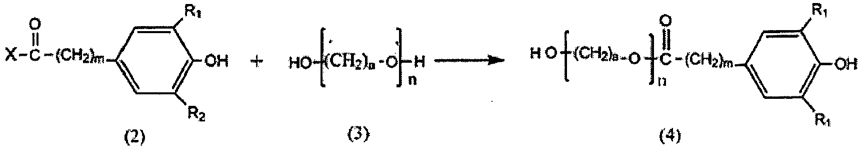

The compound (2) has the 3,5-methyl propionate structure, wherein X can be a halogen, for example, a chloride or bromide, hydroxy or alkoxy, for example an alkoxy with less than 4 carbons such as a methoxy group or an ethoxy group;
R1 and R2 can be mutually the same or different C1∼C18 straight-chain or branched-chain alkyl groups, the branched-chain alkyl groups comprises a secondary and tertiary carbon, for example, are methyl, ethyl, propyl, isopropyl, n-butyl, sec-butyl, iso-butyl, tert-butyl, 2-ethylbutyl, n-pentyl, isopentyl, 1-methyl-pentyl, 1,3-dimethyl-butyl, n-hexyl, 1-methyl-hexyl, n-pentyl, isoheptyl, 1,1,3,3-tetramethyl-butyl, 1-methyl-pentyl, 3-methyl-pentyl, n-octyl, 2-ethyl-hexyl, 1,1,3-trimethyl-hexyl, 1,1,3,3-tetramethyl-pentyl, nonyl, decyl, undecyl, 1-methyl- undecyl, dodecyl, 1,1,3,3,5,5-hexamethyl-hexyl, tridecyl, tetradecyl, pentadecyl, hexadecyl, heptadecyl or octadecyl, and preferably are methyl and tert-butyl;
In addition, R1 and R2 can be mutually the same or different C5∼C18 cycloalkyl groups, for example, cyclopentyl, cyclohexyl, cyclopentyl, cyclooctyl, cyclononyl, cyclodecyl, cycloundecyl or cyclododecyl, and preferably are cyclohexyl; m is 1 or 2; the compound (2) and preferably is methyl 3-(3,5-di-tert-butyl-4-hydroxyphenyl)propionate.

The compound (3) has the poly alkylehe glycol structure, wherein a is an integer of 2 to 6, for example, polyethylene glycol, poly propylene glycol, polybutylene glycol or polyhexylene glycol, and preferably is polyethylene glycol; n is an integer of 2 to 500, for example, the PEG200, 300, 400, 540, 600, 1000, 1450, 3350, 4000, 6000, 8000 and 20000 in CARBOWAX™ series.

The esterification can be carried out the reactants in a molten state without solvent.

The catalyzers can be organic sulfonic acid, for example, methanesulfonic acid, ethanesulfonic acid, trifluoromethanesulfonic acid, trichloromethanesulfonic acid, 4-chlorolbenzenesulfonic acid, 2,4-dinitrobenzenesulfonic acid, para-methylbenzenesulfonic acid and dodecylbenzene sulfonic acid, and preferably is para-methylbenzenesulfonic acid.

Transesterification is a common equilibrium reaction. In order to cause the equilibrium biased towards the reaction product side, byproducts are continuously removed from the reaction system during the process. A more convenience way is to vaporize alcohol byproducts with lower carbon number and low boiling point such as methanol during the transesterification reaction; also adding the reactant concentration is effective to cause the equilibrium biased towards the reaction product side. According to the present invention, the compound (3) proportion is increased for causing the equilibrium biased towards the reaction product side and further it facilitate the purpose to make the intermediate (4) from mono-substituted group transesterification become the main product.

The intermediate (4) can be separated and purified, extracted via general methods such as washing with water, organic solvent extraction, fractional distillation or column chromatography, and organic solvents used in the column chromatography preferably are n-hexane, ethyl acetate or a mixed solution of n-hexane and ethyl acetate.

The second step is that, under the catalyzation with catalyzers, the intermediate (6) is obtained by partial transesterification of the intermediate (4) and the compound (5).

The compound (5) is one of the mono-alkene polybasic acids, and can be mono-alkene dibasic acid (5-1), mono-alkene tribasic acid (5-2), mono-alkene tetrabasic acid (5-3) or cyclic mono-alkene anhydrides (5-4), wherein mono-alkene dibasic acid (5-1) comprises cis-(5-1a), trans- (5-1b) or gem- (5-1c) isomers; the above mentioned mono-alkene polybasic acids is the carboxylic acid group source of the antioxidants comprising 3,5-methyl propionate groups / poly alkylene glycol bridges / carboxylic acid groups according the present invention.

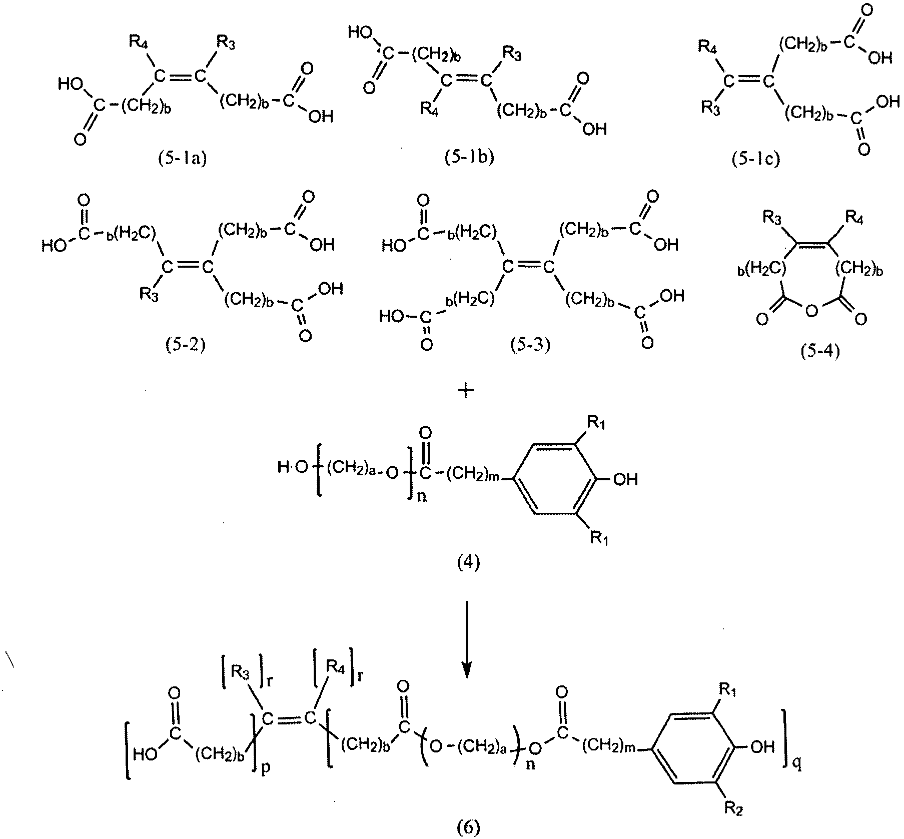

In (5-1a), (5-1b), (5-1c) and (5-4), where b is one or a combination of any two of 0, 1, 2, 3 and 4;
In (5-2), where b is one or a combination of any two or any three of 0, 1, 2, 3 and 4; In (5-3), where b is one or a combination of any two, any three or any four of 0, 1, 2, 3 and 4;
R3 and R4 can be mutually the same or different C1∼C6 straight-chain or branched-chain alkyl groups and hydrogen, the straight-chain group is methyl, ethyl, propyl, n-butyl, n-pentyl or n-hexyl, the branched-chain alkyl group is isopropyl, sec-butyl, iso-butyl, tert-butyl, 2-ethylbutyl, isopentyl,1-methyl-pentyl or 1,3-dimethyl-butyl.

The cis-mono-alkene dibasic acid (5-1a), for example, is (2Z)-2-butenedioic acid, (5Z)-5- decenedioic acid, (4Z)-4- decenedioic acid, (3Z)-3-heptenedioic acid, 2-octyl-(2Z)-2-pentenedioic acid, 2-butyl-(2Z)-2-pentenedioic acid, 4-methyl-(3Z)-3-heptenedioic acid, 2,3-diethyl-(2Z)-2-butenedioic acid and 2,3-dimethyl-(2Z)-2-butenedioic acid, and preferably is (2Z)-2-butenedioic acid.

The trans-mono-alkene dibasic acid (5-1b), for example, is (2E)-2-butenedioic acid, (5E) -5- decenedioic acid, (4E)-4- decenedioic acid, (3E)-3-heptenedioic acid, 2-octyl-(2E)-2-pentenedioic acid, 2-butyl-(2E)-2-pentenedioic acid, 4-methyl-(3E)-3-heptenedioic acid, 2,3-diethyl-(2E)-2-butenedioic acid and 2,3-dimethyl-(2E)-2-butenedioic acid, and preferably is (2E)-2-butenedioic acid.

The gem mono-alkene dibasic acid (5-1c) is, for example, 2-methylene-pentanedioic acid, 4-methylene-nonanedioic acid, 5-methylene-nonanedioic acid, 2-(1-methylheptylidene)-propanedioic acid, 2-(1-methylethylidene)-pentanedioic acid, 2-(1,3-dimethyl-1-buten-1-yl)-butanedioic acid, (1,3-dimethylbutylidene)- butanedioic acid, 2-heptylidene-(2Z)- butanedioic acid, 2-heptylidene-(2E)- butanedioic acid, 2-hexylidene-(2Z)- butanedioic acid, 2-hexylidene-(2E)- butanedioic acid and 4-ethylidene-heptanedioic acid, and preferably is 2-methylene-pentanedioic acid.

The mono-alkene tribasic acid (5-2) is, for example, 3-(carboxymethyl)-2-pentenedioic acid, 2-pentene-1,3,5-tricarboxylic acid, (2E)-2-pentene-1,2,5-tricarboxylic acid, (2Z)-2-pentene-1,2,5-tricarboxylic acid, (2E)-2-pentene-1,2,3-tricarboxylic acid, (2Z)-2-pentene-1,2,3-tricarboxylic acid, (3E)-3-dodecene-1,3,4-tricarboxylic acid, (3Z)-3-dodecene-1,3,4-tricarboxylic acid and 3-pentene-1,3,4-tricarboxylic acid, and preferably is 3-(carboxymethyl)-2-pentenedioic acid.

The mono-alkene tetrabasic acid (5-3) is , for example, 1,1,2,2-ethenetetracarboxylic acid, 1-propene-1,1,2,3-tetracarboxylic acid and 2-butene-1,2,3,4-tetracarboxylic acid, and preferably is 1,1,2,2-ethenetetracarboxylic acid.

The cyclic mono-alkene anhydride is, for example, 2,5-furandione (maleic anhydride), 3-methyl-2,5-furandione, 3,4-dimethyl-2,5-furandione, 3-ethyl-4-methyl-2,5-furandione, 3-propyl-2,5-furandione, 3,4-dipropyl-2,5-furandione, 3-methyl-4-(1-methylethyl)-2,5-furandione, 3-methyl-4-(2-methylpropyl)-2,5-furandione, 3,4-dihexyl-2,5-furandione, 2H-pyran-2,6(3H)-dione (glutaconic anhydride), 4-methyl-2H-pyran-2,6(3H)-dione (3- methyl glutaconic anhydride), 3,6-dihydro-2,7-oxepindione, 3,6-dihydro-4-methyl-2,7-oxepindione, 3,4,7,8-tetrahydro-2,9-oxonindione, and preferably is 2,5-furandione.

The esterification can be carried out the reactants in a molten state without solvent.

The applicable catalyzer can be organic sulfonic acid, for example, methanesulfonic acid, ethanesulfonic acid, trifluoromethanesulfonic acid, trichloromethanesulfonic acid, 4-chlorolbenzenesulfonic acid, 2,4-dinitrobenzenesulfonic acid, para-methylbenzenesulfonic acid and dodecylbenzene sulfonic acid, and preferably is para-methylbenzenesulfonic acid.

The molar proportion of the intermediate (4) and the compound (5) varies depending on the structure of the compound (5) and the resulting intermediate (6).

When the reactants are mono-alkene tribasic acid (5-2) or mono-alkene tetrabasic acid (5-3), the intermediate (6) mixtures, which comprise main components of 3,5-methyl propionate / poly alkylene glycol groups or carboxylic acid groups, are obtained via regulating the proportion of intermediate (4) and mono-alkene tribasic acid (5-2) or mono-alkene tetrabasic acid (5-3). The higher the degree of esterification is, the percentage of the compound (5) coupling to 3,5-methyl propionate / poly alkylene glycol groups is higher, and the extent of molecular weight increase is higher. The lower the degree of esterification is, the unreacted and residual carboxylic acid groups of the compound (5) are more and, and the extent of molecular weight increase is lower. The main components of the intermediate (6) mixtures are primarily quantified via GPC.

The intermediate (6) mixtures comprising the main components of 3,5-methyl propionate / poly alkylene glycol groups have preferred oxidation resistance but poor water solubility; and The intermediate (6) mixtures comprising the main components of carboxylic acid groups has preferred water solubility but poor oxidation resistance.

In the final step, the aqueous solution of the compound (1) is obtained firstly by adding alkaline solutions to adjust pH of the intermediate (6) to a neutral condition for carrying out a sulfonation of addition reaction to double bond with sulfite agents, and then the saponification reaction is carried out under the basic conditions, where the applicable sulfite agents include sodium sulfite or sodium bisulfite, and the applicable alkaline solutions include sodium hydroxide or potassium hydroxide aqueous solution.

The antioxidant of the present invention has characteristic such as low volatility and high water solubility etc. which are applicable to industrial aqueous phase systems. The antioxidant of the present invention has a surfactant (emulsifier) characteristic useful in-the industrial application of oil-soluble antioxidants in aqueous phase systems.

### DETAILED DESCRIPTION OF THE INVENTION

The technical contents and detailed description of the present invention are described thereinafter according to a preferable embodiment, being not used to limit its executing scope.

### (Embodiment 1)

### disodium

### (Z)-1-(3,5-di-tert-butyl-4-hydroxyphenyl)-3,17-dioxo-19-sulfonato-4,7,10,13,16-pentaoxaico san-20-oate

20.0g of methyl 3-(3,5-di-tert-butyl-4-hydroxyphenyl)propionate, 23.9g PEG200 and 3.0g para-methylbenzenesulfonic acid are put into a reaction bottle with a mechanical stirrer, thermostat and reflux condenser. The reactants are stirred for 4 hours under 125∼130°C then cooled to room temperature under nitrogen atmosphere. The reaction mixtures are dissolved via 250ml toluene added, washed several times (for removing residual PEG200 and para-methylbenzenesulfonic acid), the dehydrated with anhydrous sodium sulfate and filtered.

Next, 4.8g maleic anhydride and 3.0g para-methylbenzenesulfonic acid is added. The reactants are stirred under nitrogen atmosphere and 105∼110°C, and regularly sampled for measuring acid values. When the acid values are stable and less than 15, the reaction temperature is lowered to stop the reaction. The reaction temperature is further lowered to room temperature and 5.8g sodium hydroxide is added slowly to adjust pH to about 7. 65.0g water is divided into two portions and added respectively to extract soluble products twice, and remove the toluene layer (containing residual 3,5-methyl propionate / PEG200 mono-substituted transesterificated intermediate and 3,5-methyl propionate/ PEG200 di-substituted transesterificated intermediate). The temperature is raised to 70∼75°C, and 5.4g sodium bisulfite is added with keeping the temperature and stirring 4 hours for carrying the reaction, followed by reducing the temperature to room temperature. 0.4g 45% sodium hydroxide is added for carrying out a saponification reaction until the aqueous solution about pH=9, actual value of solid content 34.5% (theoretical value 36%).

### (Embodiment 2)

### disodium

### (Z)-1-(3,5-di-tert-butyl-4-hydroxyphenyl)-3,32-dioxo-34-sulfonato-4,7,10,13,16,19,22, 25,28,31-decaoxapentatriacontan-35-oate

20.0g methyl 3-(3,5-di-tert-butyl-4-hydroxyphenyl)propionate, 56.7g PEG400 and 3.0g para-methylbenzenesulfonic acid are put into a reaction bottle with a mechanical stirrer, thermostat and reflux condenser. The reactants are stirred for 4 hours under 125∼130°C then cooled to room temperature under nitrogen atmosphere. The reaction mixtures are dissolved via 250ml toluene added, washed several times (for removing residual PEG400 and para-methylbenzenesulfonic acid), dehydrated with anhydrous sodium sulfate and filtered.

Next, 4.2g maleic anhydride and 3.0g para-methylbenzenesulfonic acid is added. The reactants are stirred under nitrogen atmosphere and 105∼110°C, and regularly sampled for measuring acid values. When the acid values are stable and less than 13, the reaction temperature is lowered to stop the reaction. The reaction temperature is further lowered to room temperature and 5.4g sodium hydroxide is added slowly to adjust pH to about 7. 68.0g water is divided into two portions and added respectively to extract soluble products twice, and remove the toluene layer (containing residual 3,5-methyl propionate / PEG200 mono-substituted transesterificated intermediate and 3,5-methyl propionate/ PEG200 di-substituted transesterificated intermediate). The temperature is raised to 70∼75°C, and 4.7g sodium bisulfite is added with keeping the temperature and stirring 4 hours for carrying the reaction, followed by reducing the temperature to room temperature. 0.4g 45% sodium hydroxide is added for carrying out a saponification reaction until the aqueous solution about pH=9, actual value of solid content 36% (theoretical value 38%).

## Claims

1. A compound of formula (1), 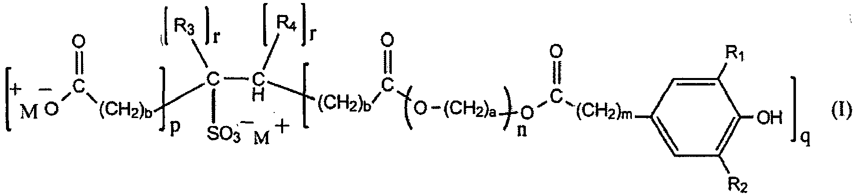 wherein,
R1 and R2 can be mutually the same or different C1∼C18 straight chain or branched-chain alkyl groups or C5∼C12 cycloalkyl groups;
R3 and R4 can be mutually the same or different C1∼C6 straight chain or branched-chain alkyl groups or hydrogen;
M is an IA metal ion or a hydrogen atom;
a is an integer of 2 to 6;
b is 0, 1, 2, 3 or 4;
m is 1 or 2;
n is an integer of 2 to 500;
p is 1, 2 or 3;
q is 1, 2 or 3; and
r is 0 or 1, and p, q and r satisfying the equation p + q + 2r = 4.

2. The compound of formula (1) according to claim 1, wherein n is an integer of 5 to 300.

3. A method for preparing the compound of formula (1) of claim 1, comprising steps:
(a) compounds of 3,5-methyl propionate structure and compounds of poly alkylene glycol structure reacting to obtain an intermediate (4) of mono-substituted group transesterification structure; 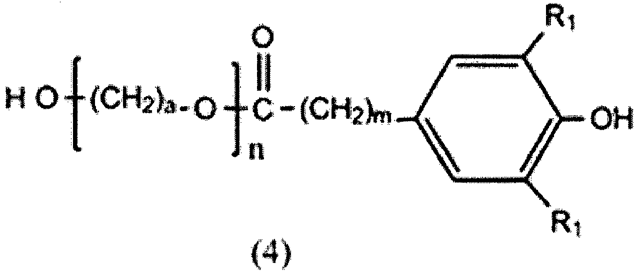 wherein,
R1 can be mutually the same or different C1∼C18 straight chain or branched-chain alkyl groups or C5∼C12 cycloalkyl groups;
a is an integer of 2 to 6;
m is 1 or 2; and
n is an integer of 2 to 500,
(b) compounds of mono-alkene polybasic acids (5) selected from the group consisting of compounds of formulae 5-1a, 5-1b, 5-1c, 5-2, 5-3 and 5-4 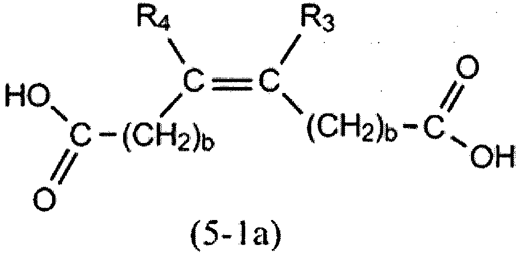 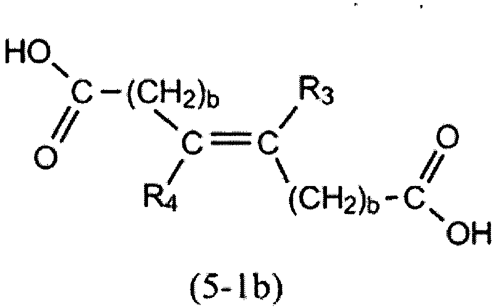 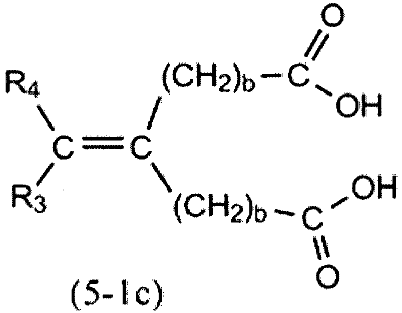 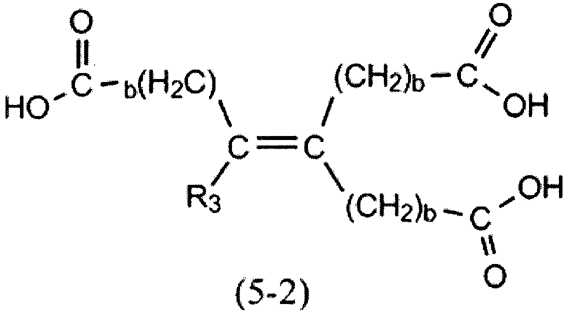 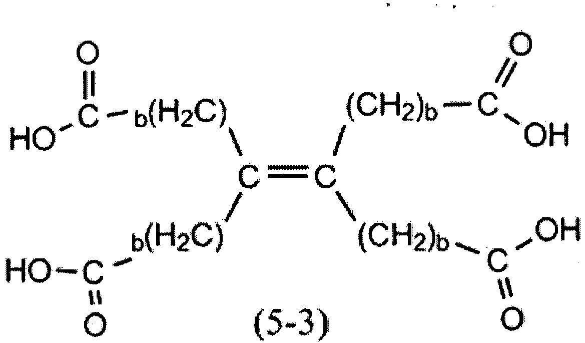 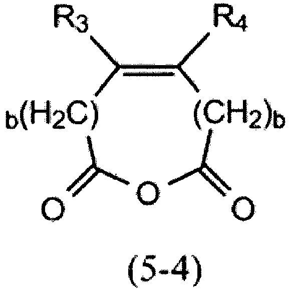 wherein,
R3 and R4 can be mutually the same or different C1∼C6 straight chain or branched-chain alkyl groups or hydrogen; and
b is 0, 1, 2, 3 or 4,
and said intermediate (4) reacting to obtain an intermediate (6); 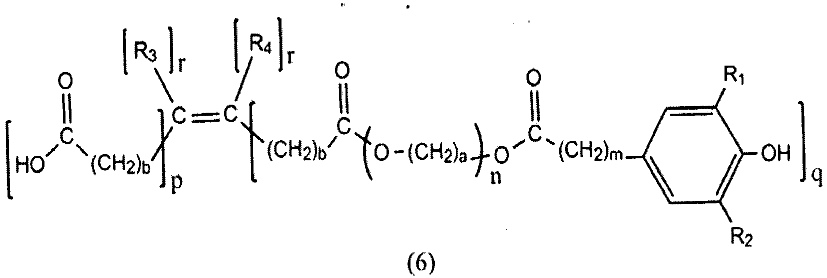 wherein,
R1 and R2 can be mutually the same or different C1∼C18 straight chain or branched-chain alkyl groups or C5∼C12 cycloalkyl groups;
R3 and R4 can be mutually the same or different C1∼C6 straight chain or branched-chain alkyl groups or hydrogen;
a is an integer of 2 to 6;
b is 0, 1, 2, 3 or 4;
m is 1 or 2;
n is an integer of 2 to 500;
p is 1, 2 or 3;
q is 1, 2 or 3; and
r is 0 or 1, and p, q and r satisfying the equation p + q + 2r = 4,
(c) said intermediate (6) being sulfonated under neutral condition, and then saponified under basic conditions for reacting to obtain an aqueous solution having the compound of formula (1).

4. The method according to claim 3, wherein said compound (5) is a mono-alkene dibasic acid, mono-alkene tribasic acid, mono-alkene tetrabasic acid or cyclic mono-alkene anhydride.

5. The method according to claim 4, wherein the mono-alkene dibasic acid of said compound (5) is a cis-, trans- or gem- isomer.

6. The method according to claim 4, wherein the cyclic mono-alkene anhydride of said compound (5) is a maleic anhydride.

7. The method according to claim 3, wherein an alkaline solution used in said saponification reaction is a sodium hydroxide aqueous solution or a potassium hydroxide aqueous solution.

8. The method according to claim 3, wherein the compound of said 3,5-methyl propionate structure is methyl 3-(3,5-di-tert-butyl-4-hydroxyphenyl)propionate.

9. The method according to claim 3, wherein the sulfonation of the step (c) is carried out with sulfite agents of sodium bisulfite or sodium sulfite.

10. The method according to claim 3, wherein the compound of said poly alkylene glycol structure is a polyethylene glycol, polypropylene glycol, polybutylene glycol or polyhexylene glycol.

11. Intermediate of formula (6) 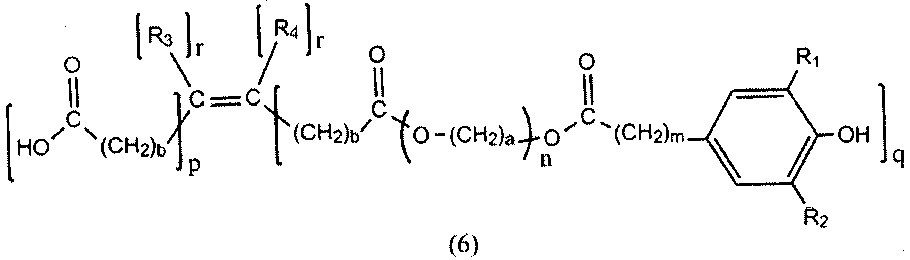 wherein,
R1 and R2 can be mutually the same or different C1∼C18 straight chain or branched-chain alkyl groups or C5∼C12 cycloalkyl groups;
R3 and R4 can be mutually the same or different C1∼C6 straight chain or branched-chain alkyl groups or hydrogen;
a is an integer of 2 to 6;
b is 0, 1, 2, 3 or 4;
m is 1 or 2;
n is an integer of 2 to 500;
p is 1, 2 or 3;
q is 1, 2 or 3; and
r is 0 or 1, and p, q and r satisfying the equation p + q + 2r = 4.

## Patentansprüche

1. Verbindung der Formel (1): 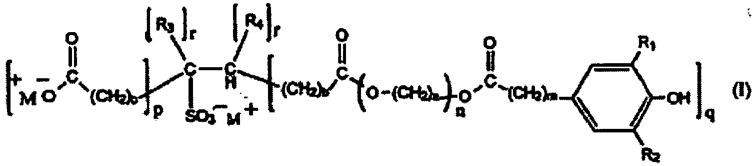 wobei
R₁ und R₂ gleiche oder verschiedene geradkettige oder verzweigte C₁- bis C₁₈-Alkylgruppen oder C₅- bis C₁₂-Cycloalkylgruppen sein können;
R₃ und R₄ gleiche oder verschiedene geradkettige oder verzweigte C₁- bis C₆-Alkylgruppen oder Wasserstoff sein können;
M ein IA-Metallion oder ein Wasserstoffatom ist;
a eine ganze Zahl von 2 bis 6 ist;
b = 0, 1, 2, 3 oder 4 ist;
m = 1 oder 2 ist;
n eine ganze Zahl von 2 bis 500 ist;
p = 1, 2 oder 3 ist;
q = 1, 2 oder 3 ist; und
r = 0 oder 1 ist und p, q und r die Gleichung p + q + 2r = 4 erfüllen.

2. Verbindung der Formel (1) gemäß Anspruch 1, wobei n eine ganze Zahl von 5 bis 300 ist.

3. Verfahren zur Herstellung der Verbindung der Formel (1) gemäß Anspruch 1, umfassend die Schritte:
(a) Umsetzen von Verbindungen mit 3,5-Methylpropionat-Struktur mit Verbindungen mit Polyalkylenglycol-Struktur, wobei man ein Zwischenprodukt (4) mit monosubstituierter Gruppenumesterungsstruktur erhält: 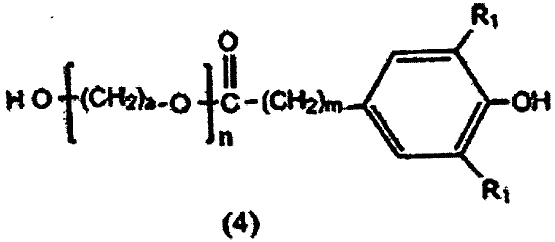 wobei
es sich bei R₁ um gleiche oder verschiedene geradkettige oder verzweigte C₁- bis C₁₈-Alkylgruppen oder C₅- bis C₁₂-Cycloalkylgruppen handeln kann; a eine ganze Zahl von 2 bis 6 ist;
m = 1 oder 2 ist; und
n eine ganze Zahl von 2 bis 500 ist.
(b) Umsetzen von Verbindungen von mehrwertigen Monoalkensäuren (5), die aus der Gruppe ausgewählt sind, die aus Verbindungen der Formeln 5-1a, 5-1b, 5-1c, 5-2, 5-3 und 5-4 besteht: 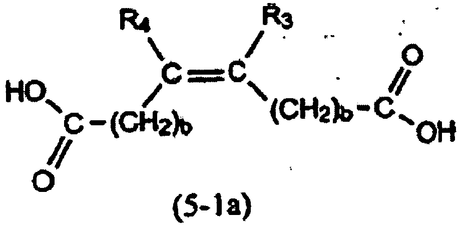 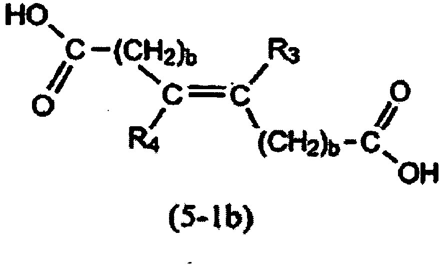 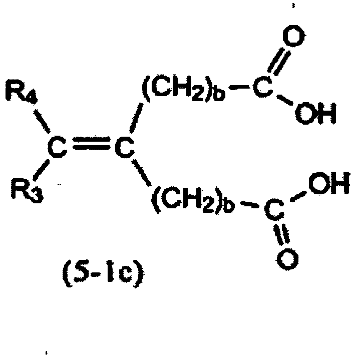 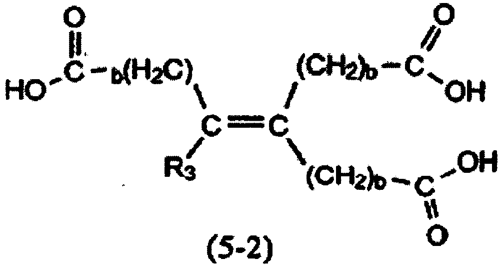 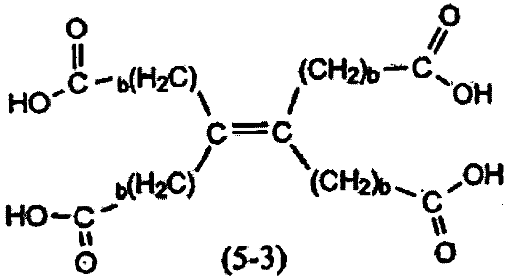 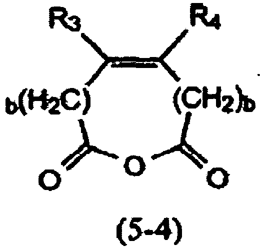 wobei
R₃ und R₄ gleiche oder verschiedene geradkettige oder verzweigte C₁- bis C₆-Alkylgruppen oder Wasserstoff sein können; und
b = 0, 1, 2, 3 oder 4 ist;
mit dem Zwischenprodukt (4), wobei man ein Zwischenprodukt (6) erhält: 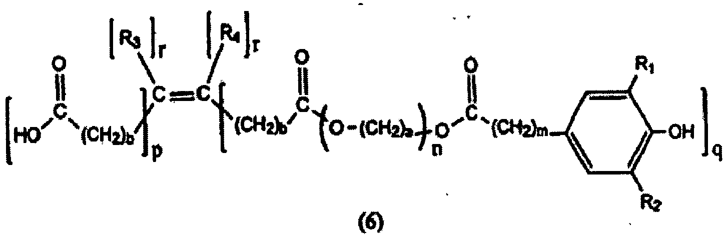 wobei
R₁ und R₂ gleiche oder verschiedene geradkettige oder verzweigte C₁- bis C₁₈-Alkylgruppen oder C₅- bis C₁₂-Cycloalkylgruppen sein können;
R₃ und R₄ gleiche oder verschiedene geradkettige oder verzweigte C₁- bis C₆-Alkylgruppen oder Wasserstoff sein können;
a eine ganze Zahl von 2 bis 6 ist;
b = 0, 1, 2, 3 oder 4 ist;
m = 1 oder 2 ist;
n eine ganze Zahl von 2 bis 500 ist;
p = 1, 2 oder 3 ist;
q = 1, 2 oder 3 ist; und
r = 0 oder 1 ist und p, q und r die Gleichung p + q + 2r = 4 erfüllen;
(c) Sulfonieren des Zwischenprodukts (6) unter neutralen Bedingungen und dann Verseifen unter basischen Bedingungen zur Umsetzung, wobei man eine wässrige Lösung mit der Verbindung der Formel (1) erhält.

4. Verfahren gemäß Anspruch 3, wobei die Verbindung (5) eine zweiwertige Monoalkensäure, eine dreiwertige Monoalkensäure, eine vierwertige Monoalkensäure oder ein cyclisches Monoalkenanhydrid ist.

5. Verfahren gemäß Anspruch 4, wobei die zweiwertige Monoalkensäure von Verbindung (5) ein cis-, trans- oder gem-Isomer ist.

6. Verfahren gemäß Anspruch 4, wobei das cyclische Monoalkenanhydrid von Verbindung (5) ein Maleinsäureanhydrid ist.

7. Verfahren gemäß Anspruch 3, wobei die alkalische Lösung, die bei der Verseifungsreaktion verwendet wird, eine Natronlauge oder Kalilauge ist.

8. Verfahren gemäß Anspruch 3, wobei es sich bei der Verbindung mit der 3,5-Methylpropionat-Struktur um Methyl-3-(3,5-di-tert-butyl-4-hydroxy-phenyl)propionat handelt.

9. Verfahren gemäß Anspruch 3, wobei die Sulfonierung in Schritt (c) mit den Sulfit-Reagentien Natriumhydrogensulfit oder Natriumsulfit durchgeführt wird.

10. Verfahren gemäß Anspruch 3, wobei die Verbindung mit der Polyalkylen-glycol-Struktur ein Polyethylenglycol, Polypropylenglycol, Polybutylen-glycol oder Polyhexylenglycol ist.

11. Zwischenprodukt der Formel (6): 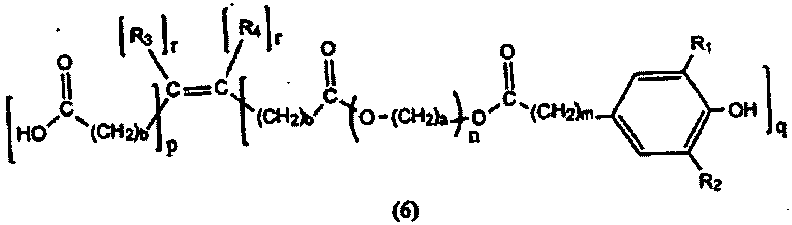 wobei
R₁ und R₂ gleiche oder verschiedene geradkettige oder verzweigte C₁- bis C₁₈-Alkylgruppen oder C₅- bis C₁₂-Cycloalkylgruppen sein können;
R₃ und R₄ gleiche oder verschiedene geradkettige oder verzweigte C₁- bis C₆-Alkylgruppen oder Wasserstoff sein können;
a eine ganze Zahl von 2 bis 6 ist;
b = 0, 1, 2, 3 oder 4 ist;
m = 1 oder 2 ist;
n eine ganze Zahl von 2 bis 500 ist;
p = 1, 2 oder 3 ist;
q = 1, 2 oder 3 ist; und
r = 0 oder 1 ist und p, q und r die Gleichung p + q + 2r = 4 erfüllen.

## Revendications

1. Composé de formule (1) : 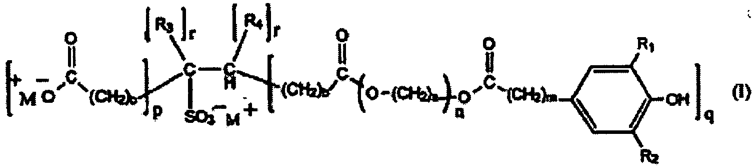 où
R1 et R2, identiques ou différents, peuvent être des groupes alkyle linéaires ou ramifiés en C₁ à C₁₈ ou des groupes cycloalkyle en C₅ à C₁₂,
R3 et R4, identiques ou différents, peuvent être des groupes alkyle linéaires ou ramifiés en C₁ à C₆ ou hydrogène,
M est un ion métallique IA ou un atome d'hydrogène,
a est un nombre entier de 2 à 6,
b est 0, 1, 2, 3 ou 4,
m est 1 ou 2,
n est un nombre entier de 2 à 500,
p est 1, 2 ou 3,
q est 1, 2 ou 3, et
r est 0 ou 1, et p, q et r satisfont l'équation p + q + 2r = 4.

2. Composé de formule (1) selon la revendication 1, dans lequel n est un nombre entier de 5 à 300.

3. Procédé pour préparer le composé de formule (1) selon la revendication 1, comprenant les étapes consistant à :
(a) faire réagir des composés de structure propionate de 3,5-méthyle et des composés de structure polyalkylène glycol pour obtenir un intermédiaire (4) de structure de transestérification de groupe monosubstitué : 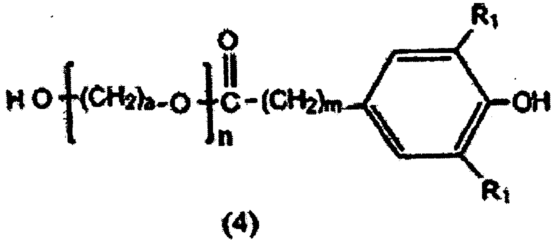 où
R1, identiques ou différents, peuvent être des groupes alkyle linéaires ou ramifiés en C₁ à C₁₈ ou des groupes cycloalkyle en C₅ à C₁₂,
a est un nombre entier de 2 à 6,
m est 1 ou 2, et
n est un nombre entier de 2 à 500,
(b) faire réagir des composés de monoalkène-acides polybasiques (5) choisis dans le groupe consistant en des composés de formule 5-la, 5-1b, 5-1c, 5-2, 5-3 et 5-4 : 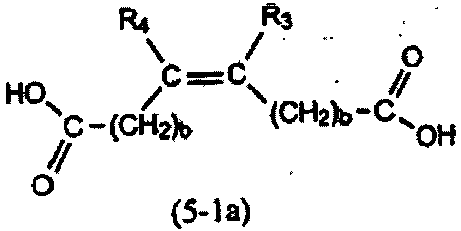 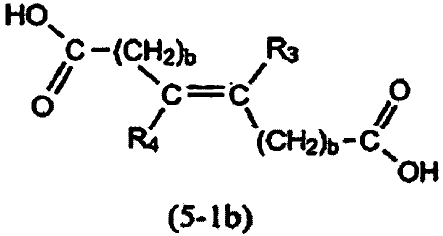 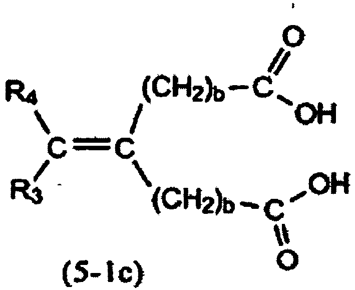 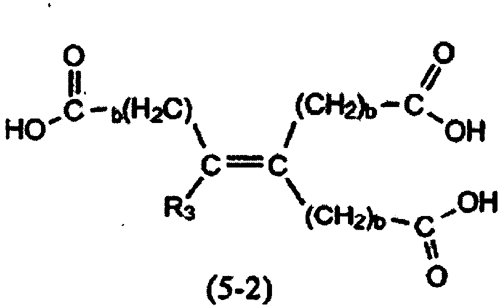 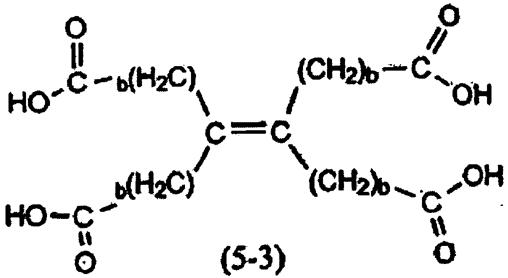 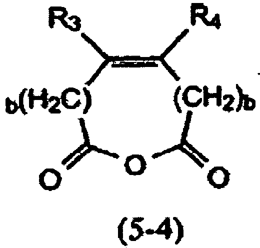 où
R3 et R4, identiques ou différents, peuvent être des groupes alkyle linéaires ou ramifiés en C₁ à C₆ ou hydrogène, et
b est 0, 1, 2, 3 ou 4,
avec l'intermédiaire (4) pour obtenir un intermédiaire (6) : 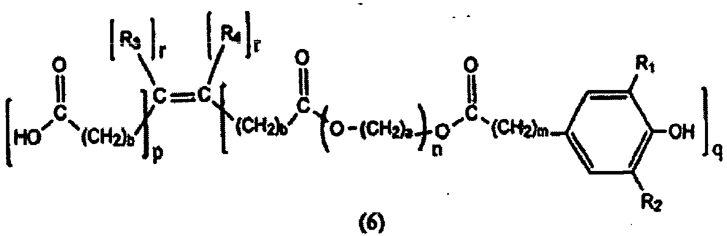 où
R1 et R2, identiques ou différents, peuvent être des groupes alkyle linéaires ou ramifiés en C₁ à C₁₈ ou des groupes cycloalkyle en C₅ à C₁₂, R3 et R4, identiques ou différents, peuvent être des groupes alkyle linéaires ou ramifiés en C₁ à C₆ ou hydrogène,
a est un nombre entier de 2 à 6,
b est 0, 1, 2, 3 ou 4,
m est 1 ou 2,
n est un nombre entier de 2 à 500,
p est 1, 2 ou 3,
q est 1, 2 ou 3, et
r est 0 ou 1, et p, q et r satisfont l'équation p + q + 2r = 4,
(c) sulfoner ledit intermédiaire (6) sous une condition neutre, et puis saponifier le produit sous des conditions basiques, pour réaction pour obtenir une solution aqueuse ayant le composé de formule (1).

4. Procédé selon la revendication 3, dans lequel ledit composé (5) est un monoalkène-acide dibasique, un monoalkène-acide tribasique, un monoalkène-acide tétrabasique, ou un monoalkène-anhydride cyclique.

5. Procédé selon la revendication 4, dans lequel le monoalkène-acide dibasique dudit composé (5) est un isomère cis, trans ou gem.

6. Procédé selon la revendication 4, dans lequel le monoalkène-anhydride cyclique dudit composé (5) est un anhydride maléique.

7. Procédé selon la revendication 3, dans lequel la solution alcaline utilisée dans ladite réaction de saponification est une solution aqueuse d'hydroxyde de sodium ou une solution aqueuse d'hydroxyde de potassium.

8. Procédé selon la revendication 3, dans lequel le composé de ladite structure propionate de 3,5-méthyle est le propionate de méthyl-3-(3,5-di-tert-butyl-4-hydroxyphényle).

9. Procédé selon la revendication 3, dans lequel la sulfonation de l'étape (c) est réalisée avec des agents de sulfite d'hydrogènesulfite de sodium ou de sulfite de sodium.

10. Procédé selon la revendication 3, dans lequel le composé de ladite structure polyalkylène glycol est un polyéthylène glycol, polypropylène glycol, polybutylène glycol ou polyhexylène glycol.

11. Intermédiaire de formule (6) : 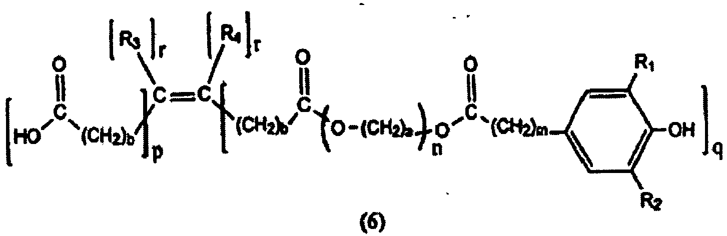 où
R1 et R2, identiques ou différents, peuvent être des groupes alkyle linéaires ou ramifiés en C₁ à C₁₈ ou des groupes cycloalkyle en C₅ à C₁₂, R3 et R4, identiques ou différents, peuvent être des groupes alkyle linéaires ou ramifiés en C₁ à C₆ ou hydrogène,
a est un nombre entier de 2 à 6,
b est 0, 1, 2, 3 ou 4,
m est 1 ou 2,
n est un nombre entier de 2 à 500,
p est 1, 2 ou 3,
q est 1, 2 ou 3, et
r est 0 ou 1, et p, q et r satisfont l'équation p + q + 2r = 4.
